# FASCICULE DE BREVET EUROPEEN

(11) **EP 0 654 261 B1**
(45) Date de publication et mention de la délivrance du brevet: **27.02.2002**
(21) Numéro de dépôt: 94402676.4
(22) Date de dépôt: 23.11.1994
(51) Int. Cl.: A61K 9/20, A61K 9/22

(54) **Composition solide mucoadhésive, thérapeutique ou hygiénique, pour administration par application sur la muqueuse buccale ou nasale**
Feste, therapeutische oder hygienische, mucoadhäsive Zusammensetzung zur Verabreichung durch Auftragen auf der Mund-schleimhaut oder der Nasen-schleimhaut
Solid, therapeutic or hygienic, mucoadhesive composition for administration by application on the buccal or nasal mucosa

(30) Priorité: 24.11.1993 FR 9314057
(43) Date de publication de la demande: 24.05.1995
(73) Titulaire: VETOQUINOL S.A., F-70200 Lure (FR)
(72) Inventeur: Jaques, Yves, F-74000 Annecy (FR); Gaillard, Claude, DK-2800 Lyngby (DK); Buri, Pierre, CH-1256 Troinex (CH); Boisrame, Bernard, F-70200 Lure (FR); Aubry, Catherine, F-70200 Lure (FR); Kaltsatos, Vassilios, F-70200 Lure (FR)
(74) Mandataire: Kédinger, Jean-Paul

(56) Documents cités:
- EP-A- 0 020 777
- EP-A- 0 107 941
- FR-A- 2 571 253
- DATABASE WPI Section Ch, Week 7616, Derwent Publications Ltd., London, GB; Class A03, AN 76-29041X & JP-A-51 026 127 (LEAD CHEM KK) 5 Mars 1976

## Description

La présente invention concerne une composition solide mucoadhésive, thérapeutique ou hygiénique, à usage humain ou vétérinaire, destinée à être administrée par application sur la muqueuse buccale ou nasale, cette composition comprenant, en mélange, un éther cellulosique gélifiable en présence d'un fluide aqueux, un homopolymère ou un copolymère de l'acide acrylique ou un sel physiologiquement acceptable de cet homopolymère ou copolymère et au moins un principe actif thérapeutique ou hygiénique.

Si le mélange d'un éther cellulosique gélifiable en milieu aqueux avec un homo- ou copolymère de l'acide acrylique est en soi doué de bonnes propriétés mucoadhésives, il est en revanche difficile, à partir de ce mélange, de réaliser industriellement des formes solides telles que des comprimés par exemple. En effet, notamment en raison de problèmes de manutention (collage) et d'écoulement médiocre de ce mélange essentiellement dus au polymère d'acide acrylique, les opérations de fabrication de comprimés sont laborieuses et il est difficile d'obtenir des comprimés de qualité constante et parfaitement reproductibles. En outre les comprimés réalisés à partir d'un tel mélange ont tendance à fortement gonfler sous l'effet des fluides aqueux (salive par exemple) ; ceci peut non seulement compromettre la cohésion de ces comprimés, mais encore provoquer, lorsque ces derniers sont appliqués sur la muqueuse buccale, une gêne dans la cavité buccale, gêne contre laquelle le sujet traité, surtout s'il s'agit d'un animal, a tendance à réagir naturellement en appliquant la langue contre le comprimé qui de ce fait subit une érosion prématurée.

EP-A-0107941 divulgue des compositions mucoadhésives destinées à être appliquées sur la muqueuse buccale et pouvant comprendre un médicament, une protéine hydrosoluble, un polyol tel qu'un éther cellulosique gélifiable en présence d'un fluide aqueux et un ester d'acide gras et/ou un polymère carboxyvinylique

L'un des buts de la présente invention est de pallier à ces inconvénients et à cet effet elle propose une composition conforme à celle définie au premier paragraphe de cette description et qui se caractérise en ce qu'elle comprend, à titre d'éther cellulosique, 15 à 25 % en poids d'au moins un élément choisi dans le groupe constitué par les éthers d'alkyle en C₁-C₄ de cellulose, les éthers d'hydroxyalkyle en C₁-C₄ de cellulose et leurs mélanges et 10 à 15 % en poids dudit homopolymère, copolymère ou sel, en ce qu'elle comprend en outre 30 à 45 % en poids de gélatine et en ce que la granulométrie dudit homopolymère, copolymère ou sel est inférieure à celle de la gélatine et est égale ou inférieure à 20 µm.

Il a en effet été constaté que par la combinaison d'éther cellulosique, d'un polymère d'acide acrylique et de gélatine dans les proportions spécifiques susmentionnées, il était possible de supprimer les inconvénients des compositions classiques antérieurement connues. Ainsi, le polymère de l'acide acrylique, en raison de sa très fine granularité, va pouvoir réaliser un enrobage physique des particules de gélatine. La séparation des particules va diminuer leur charge statique et, partant, diminuer les forces de friction et d'adhérence entre ces particules lors de la manutention, ce qui a pour effet d'améliorer l'écoulement du mélange éther cellulosique - polymère d'acide acrylique et de permettre une fabrication aisée et reproductible de la composition selon l'invention.

La formation d'un complexe par attraction des charges négatives du polymère ou copolymère de l'acide acrylique et les charges positives de la gélatine en milieu acide assure le maintien de la cohésion du comprimé et s'oppose à un gonflement excessif de la composition selon l'invention, lorsque celle-ci est amenée au contact d'un fluide aqueux.

On ajoutera qu'en dépit de la présence de la gélatine (qui est par nature un agent mucoadhésif tout à fait moyen) à une concentration relativement élevée (30-45 %) au sein de la composition selon l'invention, cette dernière conserve de manière surprenante des capacités adhésives tout à fait remarquables puisqu'elle peut être maintenue en mucoadhésion dans la cavité buccale pendant au moins 4 à 8 heures, durée tout à fait suffisante pour que ladite composition exerce de manière efficace ses effets thérapeutiques ou hygiéniques.

De plus, il est bien connu que la gélatine, lorsqu'elle est utilisée seule, forme en milieu aqueux un gel qui s'érode rapidement. Il est tout à fait inattendu que lorsqu'elle est associée (même à des teneurs aussi élevées que 45 % en poids), avec les deux autres constituants de la composition selon l'invention, elle permette l'obtention d'une composition (comprimés par exemple) présentant une cohésion tout à fait suffisante en regard de l'activité érosive des divers fluides aqueux auxquels elle est soumise in situ.

L'éther cellulosique contenu dans la composition sclon l'invention forme, en présence d'un fluide aqueux, une matrice visqueuse, insoluble et hydrophile qui assure une libération progressive et donc prolongée du ou des principes actifs de la composition. La gélatine, en raison de son caractère hydrophile, contribue à cet effet de libération prolongée, alors que le polymère de l'acide acrylique diminue le taux de relargage du ou des principes actifs. Il s'ensuit que l'association de ces trois constituants dans les proportions particulières susmentionnées, permet de moduler la vitesse de libération du ou des principes actifs pour obtenir un relargage de ces derniers pendant toute la durée de la présence de la composition in situ.

La gélatine mise en oeuvre conformément à l'invention est avantageusement une gélatine de type A qui, en solution aqueuse à 6,67 % (p/v) présente une force en gelée de 50-300 degrés Bloom, de préférence de 200-250 degrés Bloom.

La granulométrie de la gélatine mise en oeuvre est avantageusement inférieure à 250 µm, notamment inférieure à 150 µm et elle est de préférence de l'ordre de 100 µm.

Une gélatine répondant à ces caractéristiques est par exemple disponible auprès de SANOFI BIO INDUSTRIES sous la marque Gélatine AT 700 T100®.

L'éther cellulosique utilisé selon l'invention est avantageusement une hydroxypropyl cellulose ou une hydroxypropylméthyl cellulose, dont le poids moléculaire peut être situé dans la plage de 13 000 à 140 000.

Par ailleurs, l'éther cellulosique présente avantageusement une viscosité mesurée à 20° C en solution aqueuse à 2 % (p/v) de l'ordre de 10 à 75 000 mPa.s, de préférence de 10 à 15 000 mPa.s et plus préférablement de 500 à 4 000 mPa.s.

Par ailleurs, l'éther cellulosique a un degré d'éthérification qui est de préférence compris entre 0,1 et 6 ou, mieux encore, compris entre 1,2 et 3.

Enfin, la granulométrie de l'éther cellulosique sera avantageusement inférieure à 500 µm et plus préférentiellement comprise entre 150 et 250 µm.

A titre d'éthers cellulosiques, on citera par exemple ceux commercialisés par la société DOW sous les marques METHOCEL® E et METHOCEL® K et notamment sous les marques METHOCEL® E4M, METHOCEL® K15M, METHOCEL® K4M et METHOCEL® K100M.

L'homopolymère ou le copolymère de l'acide acrylique peut être utilisé sous forme de sel, notamment de sel de métal alcalin physiologiquement acceptable, comme un sel de sodium, de potassium ou d'ammonium.

La granulométrie du polymère ou copolymère de l'acide acrylique ou du sel de ce polymère ou copolymère est avantageusement de 3 à 20 µm et de préférence de 3 à 10 µm

Selon l'invention, préférence est donnée au copolymère d'acide acrylique et d'allyl sucrose, de poids moléculaire de préférence compris entre 450 000 et 4 000 000 et ayant avantageusement une viscosité mesurée à 25° C en solution aqueuse à 0,5 % (p/v) de 300 à 400 mPa.s. Un tel copolymère est disponible sous la marque CARBOPOL® 934P auprès de B.F. Goodrich Chemical Company.

Quant au principe actif de la composition selon l'invention, il sera choisi en fonction de l'affection à traiter. Il pourra s'agir notamment :
- de tous les principes actifs ayant un effet préventif ou curatif sur les pathologies buccales et nasales et en particulier les pathologies nécessitant un contact direct avec le principe actif (par exemple, antiseptiques tels que la chlorhexidine et ses sels ; antibiotiques tels que la spiramycine ; anesthésiques locaux tels que la lidocaïnc ; anti-inflammatoires tels que l'acide tolfénamique ; les agents contre les aphtes ; des enzymes comme le lysozyme, l'alpha-chymotrypsine ; des fluidifiants ; des expectorants) ;
- de tous principes actifs pour l'hygiène bucco-dentaire ; et
- de tous les principes actifs ayant une action par contact et notamment ceux susceptibles d'être dégradés lorsqu'ils sont administrés par voie orale ou parentérale (certains tonicardiaques, neuroleptiques).

La composition selon l'invention peut contenir un ou plusieurs principes actifs et leur teneur dans la composition sera suffisante pour procurer l'action thérapeutique ou hygiénique recherchée.

Outre les constituants susmentionnés, la composition selon l'invention pourra comprendre divers additifs tels qu'un véhicule (ou excipient de charge) comme l'amidon, la cellulose microcristalline, le lactose, le sorbitol, le mannitol, un phosphate ; un lubrifiant comme le stéarate de magnésium, le béhénate de glycérol, du talc, de l'huile de ricin hydrogénée ou des cires (jusqu'à 5 % en poids de la composition) ; un agent d'écoulement comme la silice colloïdale (jusqu'à 3 % en poids de la composition) ; des arômes encapsulés ou non, des facteurs de goût, des agents sucrants ou édulcorants et de manière générale toutes substances aptes à améliorer le goût, l'odeur ou l'aspect de la composition, ces arômes, facteurs, agents et substances pouvant représenter au total jusqu'à 10 % en poids de la composition.

La composition selon l'invention est de préférence utilisée sous la forme de comprimés que l'on peut obtenir par exemple, en mélangeant tous les ingrédients (de préférence dans une atmosphère d'une humidité relative non supérieure à 50 %) jusqu'à obtention d'une poudre homogène et en amenant cette dernière sous la forme de comprimés par compression directe. Les comprimés pourront notamment avoir la forme de disques plats, par exemple d'une épaisseur de l'ordre de 1-3 mm et d'un diamètre égal ou inférieur à 12 mm.

Il est néanmoins souhaitable de préparer ces comprimés par un procédé comprenant une étape de compression précédée d'une étape de granulation faisant appel à un solvant organique non-aqueux, notamment un alcool, de préférence l'alcool isopropylique. Il a en effet été constaté par des études rhéologiques que par mise en oeuvre d'une telle opération de granulation, l'écoulement du mélange obtenu à comprimer est fortement amélioré et ce, sans que le bon pouvoir adhésif des comprimés obtenus ne soit affecté. Plus précisément, ce procédé peut comprendre le mélange du ou des principes actifs, de l'éther cellulosique, de l'homopolymère ou du copolymère de l'acide acrylique ou d'un sel physiologiquement acceptable de celui-ci et de la gélatine jusqu'à obtention d'une poudre homogène, l'addition à cette poudre d'un solvant organique non-aqueux, notamment un alcool, de préférence l'alcool isopropylique, la granulation de la poudre ainsi additionnée dudit solvant, le séchage des grains résultants, un tamisage des grains séchés, l'addition éventuelle d'additifs tels qu'un excipient de charge et un lubrifiant, et une compression.

La composition selon l'invention peut également être utilisée sous la forme de granulés obtenus par la granulation de la poudre obtenue en mélangeant de façon homogène tous les ingrédients, ou encore sous la forme de cette poudre elle-même.

Par ailleurs, la composition selon l'invention peut être utilisée en la disposant en un endroit quelconque de la muqueuse buccale ou nasale [de préférence en un endroit où la salive ne stagne pas (face interne de la joue par exemple) dans le cas d'une application buccale] et mise en place par simple contact et éventuellement application d'une légère pression pendant quelques secondes.

On ajoutera que la composition bioadhésive sclon l'invention peut être retirée à tout moment pour mettre fin à la thérapie de façon urgente.

Le fluide buccal (salive) ou nasal pénètre dans la composition et hydrate les polymères, ce qui conduit à la formation d'une matrice (ou réseau) ; le fluide continuant d'affluer dans cette matrice dissout le ou les principes actifs qui sont alors progressivement libérés. Cette libération se poursuit sensiblement jusqu'à disparition de la composition ou son décollement qui, dans le cas d'un comprimé, intervient habituellement 4 à 8 heures après sa mise en place, étant précisé que les tests in vivo chez l'Homme et le chien auxquels a procédé la Demanderesse n'ont permis d'observer ni irritation, ni douleur, ni assèchement excessif au niveau de la zone d'application de la composition, ce qui démontre que cette dernière est parfaitement bien tolérée.

Les exemples ci-après sont donnés à des fins d'illustration non limitative de la présente invention.

### Exemple 1

Composition en % en poids d'un comprimé (obtenu par compression directe) pour application buccale (par exemple chez le chien ou le chat) pour l'antisepsie de la cavité buccale par exemple après une opération de détartrage ou d'arrachage de dent, ou pour un traitement anti-plaque dentaire :
. acétate de chlorhexidine 0,3
. CARBOPOL® 934P 15
. gélatine A 35
. hydroxypropylméthyl cellulose 22,5
. lactose (véhicule) 25
. silice colloïdale 0,2
. béhénate de glycérol 2

### Exemple 2

Composition en % en poids d'un comprimé (obtenu par compression directe) pour application buccale (par exemple chez le chat) contre les pathologies aphteuses ou ulcéreuses :
. nicotinamide 25
. CARBOPOL® 934P 15
. CARBOPOL 934P 5
. gélatine A 35
. hydroxypropylméthyl cellulose 22,8
. silice colloïdale 0,2
. béhénate de glycérol 2

### Exemple 3

Composition en % en poids d'un comprimé (obtenu par compression précédée d'une granulation) pour application buccale chez le chien et le chat, notamment pour le traitement des aphtes d'apparition consécutive à des abcès :
. acétate de chlorhexidine 0,3
. nicotinamide 25,20
. CARBOPOL® 934P 15,10
. gélatine A 35,25
. hydroxypropylméthyl cellulose 22,65
. huile de ricin hydrogénée 1
. béhénate de glycérol 0,5

Cette composition est obtenue en mélangeant de façon homogène de l'hydroxypropylméthyl cellulose (99g), du Carbop® 93AP (66 g), de la gélatine A (154 g), du nicotinamide (110 g) et de l'acétate de chlorhexidine (1,32g). Puis on mouille avec une quantité suffisante d'alcool isopropylique (174 g) et granule le mélange ainsi obtenu. Les grains résultants sont séchés à 25° C pendant 30 minutes puis forcés à travers une grille d'une ouverture de maille de 1 mm. Les grains ayant traversé la grille sont ensuite additionnés d'huile de ricin hydrogénée, de béhénate de glycérol et après mélange, on comprime.

### Exemple 4

Etude in vitro portant sur la cinétique de libération du principe actif.

Cette étude a été effectuée in vitro par un test de dissolution d'un comprimé conforme à celui obtenu à l'exemple 2 ci-dessus.

Plus précisément, ce test est mené dans un réacteur contenant 1 litre de milieu Sorensen à pH = 6,8 et à 37° C et équipé d'une palette tournante. Cet appareil est conforme aux normes décrites dans la Pharmacopée Européenne 2ème Edition (1991).

Des prélèvements sont effectués à intervalles réguliers dans le réacteur et analysés par spectrophotomètrie d'absorption dans l'U.V. (longueur d'onde : 262 nm).

Les résultats obtenus (moyenne pour 3 comprimés) sont comme suit :

| Temps (minutes) | % de nicotinamide libéré |
|---|---|
| 0 | 0 |
| 60 | 40 |
| 120 | 60 |
| 180 | 70 |
| 240 | 80 |

### Exemple 5

Etude in vitro portant sur le gonflement des comprimés.

Cette étude a été réalisée en plongeant un comprimé selon l'invention dans l'eau à température ambiante. Elle montre que le diamètre de ce comprimé n'a augmenté que d'un facteur de 0,25 après 6 heures d'immersion, augmentation qui ne génère que peu de gêne dans la bouche du sujet. Le volume d'hydratation in situ étant très faible, ce facteur de gonflement déterminé in vitro peut être considéré comme une valeur maximale.

### Exemple 6

Faisabilité industrielle d'un comprimé selon l'invention.

Un mélange de poudres à comprimer doit posséder de bonnes caractéristiques d'écoulement (un écoulement régulier assure régularité et homogénéité de remplissage des matrices de compression) et de bonnes caractéristiques de cohésion.

Les caractéristiques d'écoulement et d'aptitude à la compression se mesurent expérimentalement en réalisant des comprimés sur une machine industrielle de compression équipée de jauges de contrainte (mesure de pression) et :
- en vérifiant le bon remplissage de la matrice de compression par mesure du poids des comprimés, un poids homogène étant la preuve d'un bon écoulement,
- en mesurant la dureté des comprimés formés, une dureté appropriée étant signe d'une cohésion suffisante, et
- en vérifiant si l'éjection des comprimés est correcte (non adhérence au poinçon et absence de pic de collage et de grippage à l'enregistrement des pressions).

Les tests effectués sur les compositions selon l'invention, dans une machine de compression et une atmosphère à moins de 50 % d'humidité relative, ont conduit à l'obtention de comprimés sans défaut ; ils ne collent pas au poinçon, leur poids est sensiblement constant (les variations notées lors de trois fabrications de 1 à 5 kg chacune ne sont jamais supérieures à 2 % en poids) et leur dureté est conforme aux valeurs souhaitées (environ 100 N).

## Revendications

1. Composition solide mucoadhésive, thérapeutique ou hygiénique, à usage humain ou vétérinaire, destinée à être administrée par application sur la muqueuse buccale ou nasale, cette composition comprenant, en mélange, un éther cellulosique gélifiable en présence d'un fluide aqueux, un homopolymère ou un copolymère de l'acide acrylique ou un sel physiologiquement acceptable de cet homopolymère ou copolymère et au moins un principe actif thérapeutique ou hygiénique, **caractérisée en ce qu'**elle comprend, à titre d'éther cellulosique, 15 à 25 % en poids d'au moins un élément choisi dans le groupe constitué par les éthers d'alkyle en C₁-C₄ de cellulose, les éthers d'hydroxyalkyle en C₁-C₄ de cellulose et leurs mélanges et 10 à 15 % en poids dudit homopolymère, copolymère ou sel, **en ce qu'**elle comprend en outre 30 à 45 % en poids de gélatine et **en ce que** la granulométrie dudit homopolymère, copolymère ou sel est inférieure à celle de la gélatine et est égale ou inférieure à 20 µm.

2. Composition selon la revendication 1, **caractérisée en ce que** la gélatine et une gélatine de type A qui, en solution aqueuse à 6,67 % (p/v), présente une force en gelée de 50 à 300 degrés Bloom.

3. Composition selon la revendication 1 ou 2, **caractérisée en ce que** la gélatine présente une granulométrie inférieure à 250 µm et **en ce que** le polymère ou copolymère de l'acide acrylique ou le sel de ce polymère ou copolymère présente une granulométrie de 3 à 20 µm.

4. Composition selon la revendication 1, **caractérisée en ce que** l'éther cellulosique est une hydroxypropyl cellulose ou une hydroxypropylméthyl cellulose.

5. Composition selon la revendication 4, **caractérisée en ce que** l'éther cellulosique présente une viscosité mesurée à 20° C en solution aqueuse à 2 % (p/v) de 10 à 75 000 mPa.s.

6. Composition selon la revendication 4 ou 5, **caractérisée en ce que** l'éther cellulosique présente une granulométrie inférieure à 500 µm.

7. Composition selon la revendication 1, 2 ou 4, **caractérisée en ce que** le copolymère d'acide acrylique est un copolymère d'acide acrylique et d'allyl sucrose.

8. Composition selon la revendication 7, **caractérisée en ce que** le copolymère d'acide acrylique et d'allyl sucrose présente une viscosité mesurée à 25° C en solution aqueuse à 0,5 % (p/v) de 300 à 400 mPa.s.

9. Composition selon la revendication 1, **caractérisée en ce qu'**elle se présente sous la forme de comprimés obtenus par compression, cette dernière étant précédée d'une étape de granulation au moyen d'un solvant organique non aqueux.

## Claims

1. Solid mucoadhesive therapeutic or hygienic composition, for human or veterinary use, intended to be administered by application to the buccal or nasal mucous membrane, this composition comprising, in a mixture, a cellulosic ether gelifiable in the presence of an aqueous liquid, a homopolymer or copolymer of acrylic acid or a physiologically acceptable salt of that homopolymer or copolymer, and at least one therapeutic or hygienic active constituent, **characterized in that** it comprises, as cellulosic ether, 15 to 25 % by weight of an element selected from the group constituted by C₁-C₄ alkyle ethers of cellulose, C₁-C₄ hydroxyalkyl ethers of cellulose and their mixtures, and 10 to 15 % by weight of the said homopolymer, copolymer or salt, **in that** it also comprises 30 to 45 % by weight of gelatin and **in that** the particle size of said homopolymer, copolymer and salt is less than that of the gelatin and is less than or equal to 20 µm.

2. Composition according to claim 1, **characterized in that** the gelatin is a gelatin of the type A, which, in a 6.67 % (w/v) aqueous solution, has a gel strength of 50 to 300 degrees Bloom.

3. Composition according to claim 1 or 2, **characterized in that** the gelatin has a particle size of less than 250 µm, and **in that** the polymer or copolymer of acrylic acid or the salt of this polymer or copolymer has a particle size of 3 to 20 µm.

4. Composition according to claim 1, **characterized in that** the cellulosic ether is a hydroxypropyl cellulose or a hydroxypropylmethyl cellulose.

5. Composition according to claim 4, **characterized in that** the cellulosic ether has a viscosity measured at 20° C in a 2 % (w/v) aqueous solution of 10 to 75,000 mPa.s.

6. Composition according to claim 4 or 5, **characterised in that** the cellulosic ether has a particle size of less than 500 µm.

7. Composition according to claim 1, 2 or 4, **characterised in that** the copolymer of acrylic acid is a copolymer of acrylic acid and allyl sucrose.

8. Composition according to claim 7, **characterised in that** the copolymer of acrylic acid and allyl sucrose has a viscosity measured at 25°C in a 0.5 % (w/v) aqueous solution of 300 to 400 mPa.s.

9. Composition according to claim 1, **characterised in that** it is in the form of tablets obtained by compression, this latter stage being preceded by a granulation stage by means of an organic non-aqueous solvent.

## Patentansprüche

1. Feste, mucoadhäsive therapeutische oder hygienische Zusammensetzung zur menschlichen oder tierärztlichen Verwendung, die dazu bestimmt ist, durch Auftragung auf die Mund- oder Nasenschleimhaut verabreicht zu werden, wobei diese Zusammensetzung, in Mischung, einen in Gegenwart einer wässrigen Flüssigkeit gelierfähigen Celluloseether, ein Homopolymer oder ein Copolymer von Acrylsäure oder ein physiologisch annehmbares Salz dieses Homopolymers oder Copolymers und mindestens einen therapeutischen oder hygienischen Wirkstoff umfasst, **dadurch gekennzeichnet, dass** sie als Celluloseether 15 bis 25 Gew.-% von mindestens einem Bestandteil, gewählt aus der Gruppe bestehend aus den C₁-C₄-Alkylethern von Cellulose, den C₁-C₄-Hydroxyalkylethern von Cellulose und ihren Mischungen, und 10 bis 15 Gew.-% des Homopolymers, Copolymers oder Salzes umfasst, dass sie außerdem 30 bis 45 Gew.-% Gelatine umfasst und dass die Komgröße des Homopolymers, Copolymers oder Salzes kleiner ist als die Korngröße der Gelatine und gleich oder kleiner als 20 µm ist.

2. Zusammensetzung nach Anspruch 1, **dadurch gekennzeichnet, dass** die Gelatine eine Typ A-Gelatine ist, welche in wässriger Lösung mit 6,67 % (Gew./Vol.) eine Gelierkraft von 50 bis 300 Grad Bloom aufweist.

3. Zusammensetzung nach Anspruch 1 oder 2, **dadurch gekennzeichnet, dass** die Gelatine eine Korngröße von weniger als 250 µm aufweist und dass das Polymer oder Copolymer von Acrylsäure oder das Salz dieses Polymers oder Copolymers eine Korngröße von 3 bis 20 µm aufweist.

4. Zusammensetzung nach Anspruch 1, **dadurch gekennzeichnet, dass** der Celluloseether eine Hydroxypropylcellulose oder eine Hydroxypropylmethylcellulose ist.

5. Zusammensetzung nach Anspruch 4, **dadurch gekennzeichnet, dass** der Celluloseether eine Viskosität von 10 bis 75000 mPa.s, gemessen bei 20°C in wässriger Lösung mit 2% (Gew.Nol.) aufweist.

6. Zusammensetzung nach Anspruch 4 oder 5, **dadurch gekennzeichnet, dass** der Celluloseether eine Korngröße von weniger als 500 µm aufweist.

7. Zusammensetzung nach Anspruch 1, 2 oder 4, **dadurch gekennzeichnet, dass** das Copolymer von Acrylsäure ein Copolymer von Acrylsäure und Allylsucrose ist.

8. Zusammensetzung nach Anspruch 7, **dadurch gekennzeichnet, dass** das Copolymer von Acrylsäure und Allylsucrose eine Viskosität von 300 bis 400 mPa.s, gemessen bei 25°C in wässriger Lösung mit 0,5% (Gew.Nol.) aufweist.

9. Zusammensetzung nach Anspruch 1, **dadurch gekennzeichnet, dass** sie in Form von Tabletten vorliegt, die durch Kompression erhalten werden, wobei letzterer ein Granulationsschritt mittels eines nicht wässrigen organischen Lösungsmittels vorangeht.
